# EUROPÄISCHE PATENTSCHRIFT

(11) **EP 1 388 323 B1**
(45) Veröffentlichungstag und Bekanntmachung des Hinweises auf die Patenterteilung: **10.01.2007**
(21) Anmeldenummer: 03012088.5
(22) Anmeldetag: 28.05.2003
(51) Int. Cl.: A61B 17/70

(54) **Dynamische Stabilisierungseinrichtung für Knochen, insbesondere für Wirbel**
Dynamic stabilising arrangement for bones, in particular for the spinal column
Dispositif de stabilisation dynamique pour les os, notamment pour la colonne vertébrale

(30) Priorität: 09.08.2002 DE 10236691
(43) Veröffentlichungstag der Anmeldung: 11.02.2004
(73) Patentinhaber: BIEDERMANN MOTECH GmbH, 78054 VS-Schwenningen (DE)
(72) Erfinder: Biedermann, Lutz, 78048 VS-Villingen (DE); Jeszensky, Dezsö, 9000 St. Gallen (DE)
(74) Vertreter: Hofer, Dorothea

(56) Entgegenhaltungen:
- WO-A-01/08574
- WO-A1-00/15125
- WO-A1-96/32071
- DE-A- 2 821 678
- FR-A- 2 717 370
- SU-A- 848 009
- US-A- 5 733 284

## Beschreibung

Die Erfindung betrifft eine dynamische Stabilisierungseinrichtung für Knochen, insbesondere für Wirbel nach dem Oberbegriff des Patentanspruches 1.

Eine bekannte Methode der Behandlung von Bandscheibendefekten ist die operative Entnahme der defekten Bandscheibe und die Versteifung des Bandscheibenzwischenraums mit den beiden Wirbelkörpern oder nach Entnahme der defekten Bandscheibe der anschließende Einsatz einer künstlichen Bandscheibe. Im ersten Fall werden die an den versteiften Abschnitt angrenzenden Abschnitte der Wirbelsäule unnatürlich belastet und im zweiten Fall ist eine Simulierung der Eigenschaften einer natürlichen Bandscheibe noch unbefriedigend.

Aus der EP 0 669 109 B1 ist eine Vorrichtung zum Stabilisieren von benachbarten Rückenwirbeln bekannt, mit der eine geschädigte Bandscheibe und die Zwischenwirbelgelenke posterior teilentlastet werden können. Die Vorrichtung weist zwei Pedikelschrauben auf, die mit einem aus einem elastischen Kunststoff bestehenden Band jeweils fest verbunden sind und über das vorgespannte Band miteinander verbunden. Zur Übertragung von Druckkräften ist ferner ein auf das elastische Band aufgeschobener druckfester Körper zwischen den zwei Schraubenköpfen vorgesehen. Die Verwendung eines solchen Bandes ergibt jedoch keine Führungsstabilität des Bewegungssegmentes einer Wirbelsäule. Ferner ist eine Justierung der benachbarten Wirbel in ihrer Positionierung relativ zueinander nicht möglich, da das Kraftübertragungsverhalten des Bandes und des Druckelementes über die Knochenschrauben unspezifisch ist.

Aus der EP 0 516 567 B1 ist eine Vorrichtung zur Stabilisierung benachbarter Wirbel bekannt, welche ein aus einem Elastomer bestehendes Dämpfungselement aufweist, das zwischen zwei in die Wirbel eingeschraubte Monoaxialschrauben vorgesehen ist. Das Dämpfungselement weist an seinen mit den Schrauben zu verbindenden Enden jeweils einen Kugelkopf auf, welcher in jeweils ein Aufnahmeteil der Knochenschrauben einsetzbar und darin fixierbar ist. Somit ist eine geringfügige Justierung des Winkels der Knochenschraube relativ zu der Längsachse des Dämpfungselementes möglich. Es muß jedoch für jedes miteinander zu verbindende Wirbelpaar ein individuell passendes Dämpfungselement mit exakter Länge und exaktem Querschnitt gefertigt sein. Ferner ist das Kraftübertragungsverhalten des Dämpfungselements undefiniert, da es nicht nur axialen sondern auch Biege- und Torsionskräften nachgibt.

Aus der US 5 733 284 ist eine Stabilisierungsvorrichtung für Wirbel bekannt, die eine dynamische Bewegung von mittels Stäben verbundenen Wirbeln ermöglicht. Die Stäbe sind dabei über plattenartige Verankerungselemente mit den Wirbeln verbunden, wobei die plattenartigen Verankerungselemente mit jedem Wirbel an zumindest zwei verschiedenen Stellen verbunden sind.

In der DE 28 21 678 ist ein zwischen benachbarte Wirbel einsetzbares Implantat offenbart. Die Wirbel sind durch Kraftelemente verbunden, die Zug oder Druckkräfte auf die Wirbelkörper wirken lassen. Die Kraftelemente sind mit den Wirbeln mittels Verankerungsbändern mit Ösen, Zapfen und Hülsen bzw. Manschetten oder Platten verbunden.

Aus der WO 01/08574 A1 ist eine Knochenschraube bekannt, die einen im Knochen zu verankernden Schaft und einen damit verbundenen Kopf aufweist, welcher in einem Aufnahmeteil zur Aufnahme eines Stabs gehalten ist. Über eine Außenmutter mit einem im Inneren vorgesehenen hülsenförmigen Element wird Druck auf den Stab ausgeübt, welcher seinerseits auf eine Druckscheibe drückt. Damit werden Kopf und Stab blockiert.

Aus der WO 00/15125 ist eine Knochenschraube bekannt, die einen Schaft und einen damit verbundenen Kopf aufweist. Der Kopf ist in einem Verbindungsteil gelagert, welches einen Stab aufnimmt. Der Stab ist seitlich vom Kopf versetzt angeordnet und wird separat über eine Blockierschraube gesichert.

Es ist ferner bekannt, zur Fixierung der Wirbelsäule bzw. von Abschnitten der Wirbelsäule ein Implantatsystem bestehend aus einem Stab und wenigstens zwei mit dem Stab fest verbundenen Pedikelschrauben, die in entsprechende Wirbel eingeschraubt sind, vorzusehen. Damit ist jedoch keine dynamische Bewegungskontrolle der Bandscheibe oder eine dynamische Lastübernahme zum Entlasten einer Bandscheibe möglich.

Es ist Aufgabe der Erfindung eine dynamisch wirkende Stabilisierungseinrichtung für Knochen, insbesondere für benachbarte Wirbel bereitzustellen, mit der es sowohl möglich ist, die Knochen bzw. Wirbel und Zwischenwirbelgelenke zueinander zu positionieren und gleichzeitig die dazwischen angebundene Bandscheibe und Zwischenwirbelgelenke hinsichtlich der zu übertragenden Kräfte in definierter Weise zu unterstützen und teilzuentlasten.

Die Aufgabe wird gelöst durch eine Stabilisierungseinrichtung gemäß Patentanspruch 1. Weiterbildungen der Erfindung sind in den Unteransprüchen angegeben.

Weitere Merkmale und Zweckmäßigkeiten ergeben sich aus der Beschreibung von Ausführungsbeispielen anhand der Figuren. Von den Figuren zeigen:
- Fig. 1: eine schematische Seitenansicht der erfindungsgemäßen Vorrichtung in montiertem Zustand,
- Fig. 2: eine Draufsicht auf die erfindungsgemäße Vorrichtung gemäß Fig. 1;
- Fig. 3: eine Schnittdarstellung einer in der Vorrichtung verwendeten Polyaxialschraube gemäß der Linie VII in Fig. 1;
- Fig. 4: eine teilgeschnittene Darstellung durch die in der Vorrichtung verwendete Polyaxialschraube gemäß Fig. 1 entlang der Linie in Fig. 2;
- Fig. 5: eine Darstellung einer Abfolge von Schritten,
- bis Fig. 8: die die Montage der Stabilisierungseinrichtung zeigt.

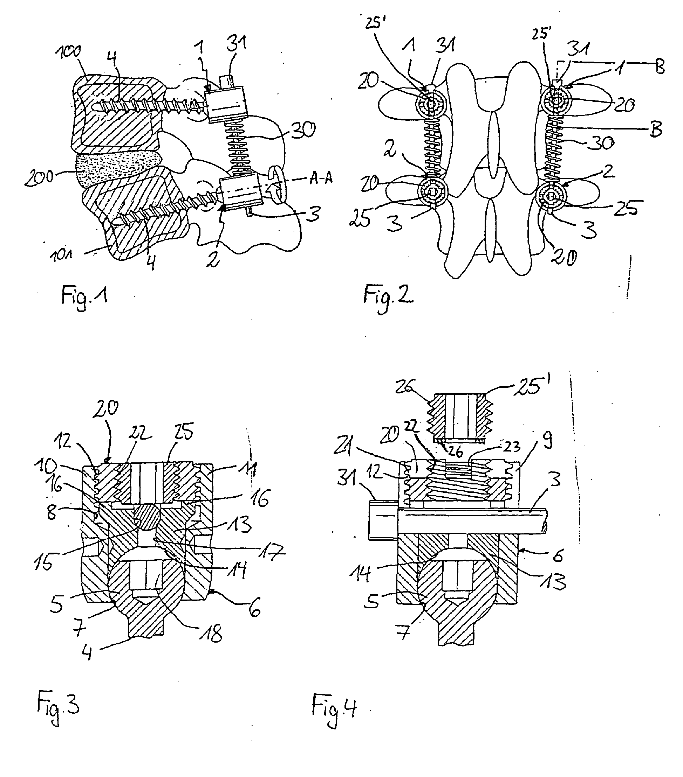

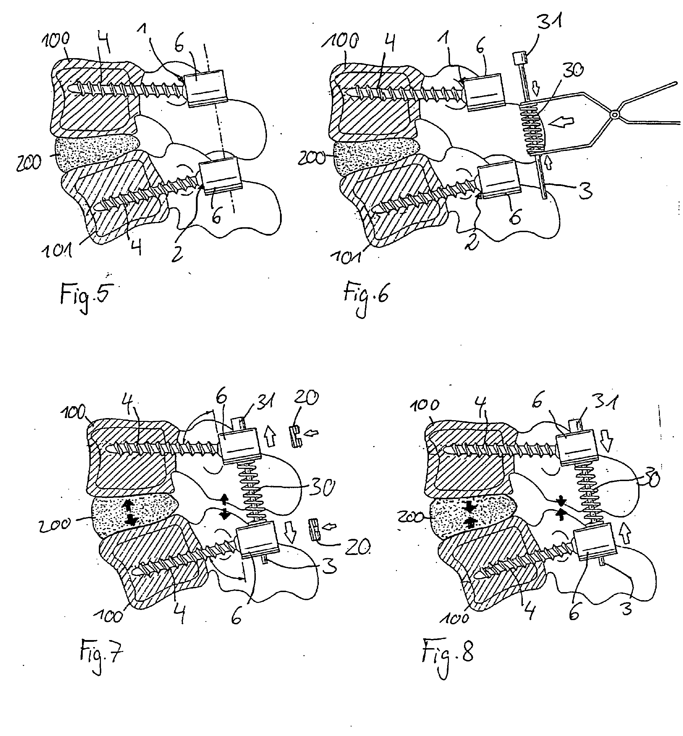

Wie insbesondere aus den Fig. 1 bis 4 ersichtlich ist, weist die Stabilisierungseinrichtung zum Stabilisieren zweier benachbarter Wirbel 100, 101 zwei polyaxiale Pedikelschrauben 1, 2 und einen diese verbindenden Stab 3 auf. Die Pedikelschrauben 1, 2 sind bevorzugt wie in Fig. 3 und 4 dargestellt ist, ausgebildet. Eine Pedikelschraube 1, 2 weist ein Schraubenelement mit einem Gewindeschaft 4 mit einem Knochengewinde und einem kugelsegmentförmigen Kopf 5 auf, der mit einem Aufnahmeteil 6 verbunden ist. Das Aufnahmeteil 6 hat an seinem einen Ende eine axialsymmetrisch ausgerichtete erste Bohrung 7, deren Durchmesser größer ist, als der des Gewindeabschnitts des Schafts 4 und kleiner als der des Kopfes 5 ist. Ferner weist es eine koaxiale zweite Bohrung 8 auf, die an dem der ersten Bohrung 7 gegenüberliegendem Ende offen ist und deren Durchmesser so groß ist, daß das Schraubenelement durch das offene Ende mit seinem Gewindeabschnitt durch die erste Bohrung 7 hindurch und mit dem Kopf 5 bis zum Grund der zweiten Bohrung führbar ist. Der Grund des Aufnahmeteils ist derart ausgebildet, daß das Schraubenelement im eingeführten und unbelasteten Zustand in dem Aufnahmeteil 6 schwenkbar ist. Das Aufnahmeteil weist ferner eine zur Mitte hin symmetrisch angeordnete U-förmige Ausnehmung auf, deren Grund zur ersten Bohrung 7 hin gerichtet ist und durch die zwei freie Schenkel 10, 11 gebildet sind. In einem Bereich angrenzend an das freie Ende weisen die Schenkel 10, 11 ein Innengewinde 12 auf.

Die Pedikelschraube beinhaltet außerdem ein Druckelement 13, welches mit einem geeigneten Außendurchmesser so ausgebildet ist, daß es in das Aufnahmeteil 6 eingeschoben werden kann. An seinem einen Ende ist eine sich zu der ersten Bohrung 7 des Aufnahmeteils 6 hin erweiternde kugelsegmentförmige Ausnehmung 14 vorgesehen, deren Kugelradius so gewählt, daß er in einem in das Aufnahmeteil eingesetzten Zustand den Kopf 5 des Schraubenelements teilweise umfaßt. In Richtung des freien Endes der Schenkel 10, 11 weist das Druckelement 13 eine U-förmige Ausnehmung 15 auf, deren Abmessungen so bemessen sind, daß in den dadurch gebildeten Kanal der Stab 3 eingelegt werden kann. Die in Richtung der Zylinderachse des Aufnahmeteils 6 gesehene Tiefe der U-förmigen Ausnehmung 15 ist größer als der Durchmesser des aufzunehmenden Stabs 3, so daß das Druckelement 13 mit seitlichen Schenkeln 16 über den eingelegten Stab 3 nach oben hervorsteht. Das Druckelement 13 weist ferner eine zentrale Bohrung 17 auf, die sich durch dieses hindurch erstreckt zum Ineingriffbringen eines Schraubwerkzeuges mit einer entsprechenden in dem Kopf 5 vorgesehenen Ausnehmung 18.

Zum Fixieren des Schraubenelements in dem Aufnahmeteil ist ein zwischen die Schenkel 10, 11 einschraubbares muffen- bzw. mutterartiges Verschlußelement 20 vorgesehen, welches ein Außengewinde 21 aufweist, das mit dem Innengewinde 12 der Schenkel zusammenwirkt und welches ferner ein Innengewinde 22 aufweist. Zum Einschrauben weist das Verschlußelement 20 ferner an seinem einen Ende radial verlaufende Einschnitte 23 auf. Die Abmessungen des Verschlußelements 20 in axialer Richtung des Aufnahmeteils, sowie die Abmessungen der freien Schenkel 10, 11 des Aufnahmeteils und die Abmessungen der zusammenwirkenden Gewinde bzw. die Höhe der freien Schenkel 16 des Druckelements sind derart bemessen, daß das Verschlußelement 20 in eingeschraubtem Zustand auf die Schenkel 16 des Druckelements eine Kraft ausübt, so daß dieses den Kopf 5 in dem Aufnahmeteil 6 blockiert.

Ferner ist eine in das Verschlußelement 20 einschraubbare Innenschraube bzw. Klemm- oder Setzschraube 25 vorgesehen, deren Außengewinde 26 mit dem Innengewinde 22 des Verschlußelements 20 zusammenwirkt. Die Abmessungen der Innenschraube 25, des Verschlußelements 20 und des Druckelements 13 sind so gewählt, daß die Innenschraube 25 in eingeschraubtem Zustand auf den eingelegten Stab 3 drückt.

In Fig. 4 zeigt einen Schnitt durch die Pedikelschraube 1 gemäß den Figuren 1 und 2. Die Pedikelschraube 1 unterscheidet sich von der Pedikelschraube 2 durch die Ausbildung der Innenschraube. Wie aus Fig. 4 ersichtlich ist, weist die Innenschraube 25' der Pedikelschraube 1 an ihrer dem Stab zugewandten Seite einen Gleitboden 26 aus einem Gleitmaterial auf, um im Betrieb ein reibungsarmes Gleiten des Stabes zu ermöglichen. Als Gleitmaterial wird beispielsweise ein hochmolekulares Polyethylen eingesetzt.

Wie aus den Figuren 1 und 2 ersichtlich ist, beinhaltet die Stabilisierungseinrichtung ferner ein Federelement 30, welches zwischen den zwei über den Stab miteinander verbundenen Pedikelschrauben 1, 2 vorgesehen ist. Das Federelement 30 ist als eine Schraubenfeder mit einem Durchmesser, der etwas größer ist, als der Durchmesser des Stabes 3, ausgebildet, so daß die Schraubenfeder auf den Stab 3 aufschiebbar ist. Die Länge der Schraubenfeder in axialer Richtung ist angepaßt an die Größenordnung des mit dem Stab zwischen den zwei Pedikelschrauben zu überbrückenden Abstandes der benachbarten Wirbel. Ferner ist die Länge der Schraubenfeder und die Federkraft so bemessen, daß eine für ein vorliegendes Funktionsdefizit der Bandscheibe gewünschte Extensions- bzw. Kompressionswirkung mit der Feder erzielbar ist. Die Feder ist bevorzugt mit einem abriebfesten Material z.B. mit einem abriebfesten Kunststoff beschichtet.

Der Stab 3 weist an seinem einen Ende einen Anschlag 31, z.B. in Form einer ringförmigen Schulter auf, die einen Durchmesser hat, der größer ist, als der Durchmesser der U-förmigen Ausnehmung des Aufnahmeteils 6 und des Druckelements 13, so daß in montiertem Zustand die dem Anschlag 31 benachbarte Pedikelschraube 1 nur bis zu diesem verschiebbar ist. Bevorzugt ist der Stab mit einem Material, insbesondere mit einem geeigneten Kunststoff, beschichtet, das ein Gleiten des Stabes in dem Aufnahmeteil 6 bzw. in dem darin vorgesehenen Druckelement 13 erleichtert. Bevorzugt ist ferner auch das Druckelement 15 wenigstens einer der Pedikelschrauben mit einem die Gleitfähigkeit erhöhenden Material, z.B. einem Kunststoff beschichtet.

Im Betrieb werden, wie in Fig. 5 ersichtlich ist, zuerst die in die Aufnahmeteile 6 eingesetzten Schraubenelemente der Pedikelschrauben 1, 2 durch den Chirurgen in die einer defekten Bandscheibe 200 benachbarten Wirbel eines Patienten im entlasteten Zustand eingeschraubt und die Aufnahmeteile 6 so ausgerichtet, daß der Stab 3 einsetzbar ist. Die Druckelemente 13 sind bereits in die Aufnahmeteile eingeführt. Als nächstes wird, wie in Fig. 6 gezeigt ist, der Stab 3 mit der auf diesen aufgeschobenen Feder 30 in die Aufnahmeteile 6 eingesetzt. Der Stab 3 wird dabei so orientiert, daß der Anschlag 31 in Richtung des Kopfes des Patienten zeigt. Ferner wird die Feder 30 mittels eines Werkzeuges vorkomprimiert, um sie unter einer Vorspannung zwischen die beiden Aufnahmeteile 6 einzubringen. Im nächsten Schritt, der in Fig. 7 dargestellt ist, stellt der Chirurg die optimale winkelstellung von Schraubenelement zu Aufnahmeteil bzw. Stab für jede der Pedikelschrauben 1, 2 ein. Diese Winkelstellung wird anschließend durch Einschrauben der Verschlußelemente 20 in die Aufnahmeteile fixiert. Wie aus Fig. 3 und 4 ersichtlich ist, erfolgt die Blockierung der Winkelstellung dadurch, daß das Verschlußelement 20 eine Kraft so auf das Druckelement 13 ausübt, daß dieses den Kopf 5 in dem Aufnahmeteil in seiner Stellung fixiert. Da die Schenkel 16 des Druckelements über den eingelegten Stab 3 hinaus hervorstehen, wird durch das Einschrauben des Verschlußelementes 20 der Stab 3 nicht berührt und ist in dem Aufnahmeteil 6 jeweils noch frei verschiebbar.

Durch die Winkeleinstellung von Schraubenelement und Aufnahmeteil zueinander läßt sich ein gewünschter Keilwinkel zwischen den einander gegenüberliegenden Oberflächen der benachbarten Wirbel einstellen, um es der dazwischen befindlichen Bandscheibe zu ermöglichen, ihre natürliche Form wieder anzunehmen. Durch Verwendung von je zwei Stabilisierungseinrichtungen wie in Fig. 2 gezeigt ist, ist dabei die Einstellung des Winkels in seitlicher und frontaler Sicht unabhängig voneinander möglich. Damit kann auch die Position der Zwischenwirbelgelenke zueinander bestimmt werden.

Wie ferner aus Fig. 7 ersichtlich ist, expandiert die unter Vorspannung eingesetzte Feder 30 nach dem Einsetzen und drückt somit die Aufnahmeteile 6 auseinander. Auf einer Seite wird die Expansion durch den Anschlag 31 begrenzt. Durch die Expansion erfolgt eine Aufweitung des Zwischenwirbelraumes und der Zwischenwirbelgelenke, wodurch die Bandscheibe 200 durch Aufnahme von Flüssigkeit aus dem Zwischenwirbelraum sich ausdehnen kann und die Zwischenwirbelgelenke entlastet werden, was durch die Pfeile in Fig. 7 veranschaulicht ist. Somit kann eine beschädigte Bandscheibe ihre natürliche Form teilweise wieder annehmen.

Wie in Fig. 8 gezeigt ist, wird anschließend die Feder durch Aufeinanderzubewegen der Aufnahmeteile 6 etwas komprimiert um sie wieder unter Vorspannung zu bringen. Dadurch verkleinert sich auch der Zwischenwirbelraum und die Bandscheibe wird wieder etwas zusammengedrückt bzw. verkürzt und die Zwischenwirbelgelenke werden belastet, was durch die Pfeile in Fig. 8 dargestellt ist. In der gewünschten Endstellung wird schließlich der Stab 3 mit dem Aufnahmeteil 6 derjenigen Pedikelschraube fest verbunden, die an dem dem Anschlag gegenüberliegenden Ende des Stabes 3 positioniert ist. Die Stabfixierung erfolgt durch Einschrauben der Innenschraube 25. Bei der angrenzend an den Anschlag 31 des Stabes vorgesehenen Pedikelschraube 1 bleiben das Aufnahmeteil 6 und der Stab 3 zueinander beweglich. Die Innenschraube 25' mit dem Gleitboden 26' ermöglicht ein reibungsarmes Gleiten des Stabes.

In der in den Fig. 1 und 2 gezeigten Endstellung wirkt das System als Kraftübertragungs- und Dämpfungssystem. Die bei aufrechter Haltung des Patienten auf die Wirbelsäule einwirkenden Kräfte werden zum Teil über das System aus Pedikelschrauben, Feder und Stab übertragen, so daß die Bandscheibe entlastet wird. Ferner wirkt die Feder sowohl als Extensionselement zum Aufweiten des Zwischenwirbelraums im Ruhe- bzw. entlasteten Zustand, z.B. beim Liegen, als auch als Dämpfer zum Dämpfen von Stößen bei Belastungen, wie z.B. beim Gehen.

Das System weist den Vorteil auf, daß bei der Montage eine optimale Justierung der Knochenschrauben und des Stabes möglich ist. Durch die steife Verbindung über den Stab ist es möglich, axiale Kräfte zu übertragen und somit die Bandscheibe zu entlasten. Das System ist jedoch biege- und torsionssteif, was einen weiteren Vorteil im Hinblick auf eine präzise Kraftübertragung auf die Bandscheibe beinhaltet.

Die Erfindung ist nicht auf die Verbindung von nur zwei polyaxialen Pedikelschrauben durch einen Stab beschränkt. Wenn es erforderlich ist, können auch mehrere Wirbel miteinander verbunden sein, wobei dann eine entsprechende Anzahl von Polyaxial-Knochenschrauben vorhanden ist. Je nach gewünschter Beweglichkeit ist an geeigneter Stelle ein Anschlag auf dem Stab vorgesehen und die entsprechende angrenzende Knochenschraube relativ zum Stab verschiebbar gehalten.

Das Federelement 30 kann auch anders ausgebildet sein. Beispielsweise kann das Federelement 30 als eine Schraubenfeder ausgebildet sein, die im Inneren des Stabes vorgesehen ist. Hierzu ist der Stab zweiteilig aus zwei ineinandergesteckten Hülsen gebildet, die jeweils einen Hülsenboden aufweisen, an dem die Enden der Schraubenfeder anliegen. Das Federelement kann ferner auch als ein elastisches Rohr aus einem Polymer ausgebildet sein.

## Patentansprüche

1. Dynamische Stabilisierungseinrichtung für Knochen, insbesondere für Wirbel,
mit wenigstens einem ersten (1) und einem zweiten (2) Knochenverankerungselement, mit jeweils einem ersten, in einem Knochen zu verankernden Abschnitt (4) und einem zweiten, mit einem Stab (3) zu verbindenden Abschnitt (6), und
einem die Knochenverankerungselemente (1, 2) verbindenden Stab (3),
wobei die Knochenverankerungselemente mit dem Stab wahlweise fest oder in Richtung der Stabachse verschiebbar verbindbar sind, und wobei
ein zwischen den Knochenverankerungselementen (1, 2) angeordnetes, in Richtung der Stabachse elastisch vorspannbares Element (30) vorgesehen ist,
**dadurch gekennzeichnet, daß** wenigstens ein Knochenverankerungselement einen Schaft (4) zum Verankern im Knochen und einen mit dem Schaft verbundenen Kopf (5) sowie ein Aufnahmeteil (6), in dem der Kopf (6) gelenkig gehalten ist, zur Aufnahme des Stabes aufweist, wobei ein Druckelement (13) vorgesehen ist, das in das Aufnahmeteil eingeschoben werden kann und eine U-förmige Ausnehmung (15) für den Stab aufweist, sodass das Druckelement mit seitlichen Schenkeln (16) über den eingelegten Stab (3) hervorsteht, und ferner ein Verschlusselement (20) vorgesehen ist, das eine Kraft so auf das Druckelement (13) ausübt , dass dieses den Kopf (5) in dem Aufnahmeteil (6) in seiner Stillung unabhängig von einer Fixierung des Stabs fixiert.

2. Dynamische Stabilisierungseinrichtung nach Anspruch 1, **dadurch gekennzeichnet, daß** eines der Knochenverankerungselemente (1) mit dem Stab verschiebbar verbunden ist und daß ein Anschlag (31) zur Begrenzung der Bewegung des verschiebbarren Knochenverankerungselementes vorgesehen ist.

3. Dynamische Stabilisierungseinrichtung nach Anspruch 1 oder 2, **dadurch gekennzeichnet, daß** der Schaft (4) und das Aufnahmeteil (6) relativ zueinander in ihrer Winkelstellung unabhängig von einer Fixierung des Stabes fixierbar sind.

4. Dynamische Stabilisierungseinrichtung nach Anspruch 3, **dadurch gekennzeichnet, daß** das polyaxiale Knochenverankerungselement (1) mit dem Stab verschiebbar verbunden und dem Anschlag benachbart angeordnet ist.

5. Dynamische Stabilisierungseinrichtung nach einem der Ansprüche 1 bis 4, **dadurch gekennzeichnet, daß** eines der Knochenverankerungeelemente (2) foot mit dem Stab (3) verbunden ist.

6. Dynamische Stabilisierungseinrichtung nach einem der Ansprüche 1 bis 5, **dadurch gekennzeichnet, daß** der Stab (3) und/oder Teile eines der Knochenverankerungselemente (1, 2) mit einem Gleitmaterial beschichtet ist.

7. Dynamische Stabilisierungseinrichtung nach einem der Ansprüche 1 bis 6, **dadurch gekennzeichnet, daß** das elastische Element (30) als Feder ausgebildet ist.

8. Dynamische Stabilisierungseinrichtung nach Anspruch 7, **dadurch gekennzeichnet, daß** die Feder (30) als eine den Stab umschließende Schraubenfeder ausgebildet ist.

9. Dynamische Stabilisierungseinrichtung nach Anspruch 7, **dadurch gekennzeichnet, daß** der Stab (3) zweiteilig und hülsenförmig ausgebildet ist, und daß die Feder im Stabinneren vorgesehen ist.

10. Dynamische Stabilisierungseinrichtung nach einem der Ansprüche 1 bis 9, **dadurch gekennzeichnet, daß** die Knochenveran kerungselemente als Knochenschrauben oder Knochenhaken ausgebildet sind.

## Claims

1. A dynamic stabilization device for bones, in particular for vertebrae, with
at least first (1) and second (2) bone anchoring elements each having a first section (4) to be anchored in a bone and a second section (6) to be connected to a rod (3), and
a rod (3) connecting the bone anchoring elements (1, 2); and
wherein the bone anchoring elements are selectably connectable to the rod either rigidly or displaceable in the direction of the rod axis, and wherein
an element (30) is provided which is arranged between the bone anchoring elements (1, 2) and can be elastically biased in the direction of the rod axis,
**characterized in that** at least one bone anchoring element comprises a shank (4) for anchoring in the bone and a head (5) connected to the shank, as well as a receiving part (6) in which the head (6) is articulatedly held for receiving the rod,
wherein
a pressure element (13) is provided which can be pushed into the receiving part and has a U-shaped recess (15) for the rod, such that the pressure element protrudes above the placed-in rod (3) with lateral legs (16), and further
a locking element (20) is provided which exerts a force on the pressure element (13) in such a way that the latter fixes the head (5) in the receiving part (6) in its position independent from a fixation of the rod.

2. A dynamic stabilization device according to claim 1, **characterized in that** one of the bone anchoring elements (1) is displaceably connected to the rod and that a stop (31) for limiting the motion of the displaceable bone anchoring element is provided.

3. A dynamic stabilization device according to claim 1 or 2, **characterized in that** the shank (4) and the receiving part (6) are fixable in their angular position relative to each other independent from a fixation of the rod.

4. A dynamic stabilization device according to claim 3, **characterized in that** the polyaxial bone anchoring element (1) is displaceably connected to the rod and is located adjacent to the stop.

5. A dynamic stabilization device according to one of claims 1 to 4, **characterized in that** one of the bone anchoring elements (2) is fixedly connected to the rod (3).

6. A dynamic stabilization device according to one of claims 1 to 5, **characterized in that** the rod (3) and/or parts of one of the bone anchoring elements (1, 2) is/are coated with a gliding material.

7. A dynamic stabilization device according to one of claims 1 to 6, **characterized in that** the elastic element (30) is constructed as a spring.

8. A dynamic stabilization device according to claim 7, **characterized in that** the spring (30) is constructed as a helical spring surrounding the rod.

9. A dynamic stabilization device according to claim 7, **characterized in that** the rod (3) is constructed in two parts and sleeve-shaped and that the spring is provided in the interior of the rod.

10. A dynamic stabilization device according to one of claims 1 to 9,**characterized in that** the bone anchoring elements are constructed as bone screws or bone hooks.

## Revendications

1. Dispositif de stabilisation dynamique pour les os, en particulier pour des vertèbres, avec au moins un premier élément d'ancrage (1) et un deuxième élément d'ancrage (2), à chaque fois avec une section (4) devant être ancrée dans un os et une deuxième section (6) qui doit être reliée à une tige (3), et une tige (3) qui relie les éléments d'ancrage (1, 2) pour les os,
les éléments d'ancrage pour les os pouvant être reliés au choix de manière fixe ou dans la direction de l'axe de la tige de manière à se déplacer, et
un élément disposé entre les éléments d'ancrage (1, 2) pour les os étant prévu dans la direction de l'axe de manière à être précontraint de manière élastique,
**caractérisé en ce qu'**au moins un élément d'ancrage pour les os présente un corps (4) pour ancrer dans l'os et une tête (5) reliée au corps ainsi qu'une partie de logement (6), dans laquelle la tête (6) est maintenue de façon articulée, afin de pouvoir loger la tige, un élément de compression (13) pouvant se coulisser dans la partie de logement, est prévu et présente un évidement en forme de U (15) destiné à la tige de manière telle que l'élément de compression avec des branches (16) latérales dépasse de la tige (3) disposée, et on prévoit en outre un élément de fermeture (20), qui exerce une force telle sur l'élément de compression (13) que celui-ci fixe la tête (5) dans la partie de logement (6) dans sa position indépendamment d'une fixation de la tige.

2. Dispositif de stabilisation dynamique selon la revendication 1, **caractérisé en ce qu'**un des éléments d'ancrage (1) pour les os est relié à la tige de manière à pouvoir se déplacer, et **en ce qu'**une butée (31) est prévue pour établir une limite du mouvement de l'élément d'ancrage mobile pour les os.

3. Dispositif de stabilisation dynamique selon la revendication 1 ou 2, **caractérisé en ce que** le corps (4) et la partie de logement (6) sont fixés l'un par rapport à l'autre dans une position d'angle indépendamment d'une fixation de la tige.

4. Dispositif de stabilisation selon la revendication 3, **caractérisé en ce que** l'élément d'ancrage (1) pour les os polyaxial est relié à la tige de manière à pouvoir se déplacer et est disposé dans le voisinage de la butée.

5. Dispositif de stabilisation selon l'une quelconque des revendications 1 à 4, **caractérisé en ce que** l'un des éléments d'ancrage (2) pour les os est relié à la tige (3) de manière fixe.

6. Dispositif de stabilisation selon l'une quelconque des revendications 1 à 5, **caractérisé en ce que** la tige (3) et/ou des parties d'un des éléments d'ancrage (1, 2) pour les os est/sont recouverte(s) d'un matériau de glissement.

7. Dispositif de stabilisation selon l'une quelconque des revendications 1 à 6, **caractérisé en ce que** l'élément élastique (30) est configuré en tant que ressort.

8. Dispositif de stabilisation selon la revendication 7, **caractérisé en ce que** le ressort (30) est configuré en tant que ressort hélicoïdal qui entoure la tige.

9. Dispositif de stabilisation selon la revendication 7, **caractérisé en ce que** la tige (3) est configurée en deux partie et est en forme de douille, et **en ce que** le ressort est prévu à l'intérieur de la tige.

10. Dispositif de stabilisation selon l'une quelconque des revendications 1 à 9, **caractérisé en ce que** les éléments d'ancrage pour les os sont configurés en tant que vis pour les os ou en tant qu'agrafe pour les os.
